# EUROPÄISCHE PATENTSCHRIFT

(11) **EP 1 494 992 B1**
(45) Veröffentlichungstag und Bekanntmachung des Hinweises auf die Patenterteilung: **28.05.2008**
(21) Anmeldenummer: 03712131.6
(22) Anmeldetag: 03.04.2003
(51) Int. Cl.: C07C 69/74, C07C 69/75, C07D 321/12, A61K 31/215, A61K 31/194, A61K 31/16, A61K 31/357, A61P 3/00, A61P 19/00, A61P 21/00, A61P 25/14, A61P 25/28, A61P 35/00, A61P 43/00

(54) **CARBOCYCLISCHE UND OXACARBOCYCLISCHE FUMARSAEURE-OLIGOMERE**
CARBOCYCLIC AND OXACARBOCYCLIC FUMARIC ACID OLIGOMERS
OLIGOMERES CARBOCYCLIQUES ET OXACARBOCYCLIQUES D'ACIDE FUMARIQUE

(30) Priorität: 18.04.2002 DE 10217314
(43) Veröffentlichungstag der Anmeldung: 12.01.2005
(60) Teilanmeldung: 06006969.7
(73) Patentinhaber: Fumapharm AG, 6006 Luzern (CH)
(72) Erfinder: JOSHI, Rajendra, Kumar, CH-8047 Zürich (CH); STREBEL, Hans-Peter, CH-6006 Luzern (CH)
(74) Vertreter: Schwabe - Sandmair - Marx
(86) Internationale Anmeldenummer: PCT/EP2003/003498
(87) Internationale Veröffentlichungsnummer: WO 2003/087174

(56) Entgegenhaltungen:
- EP-A- 0 188 749
- EP-A- 0 312 697
- WO-A-00/12072
- WO-A-00/23068
- WO-A-00/30622
- WO-A-01/51047
- WO-A-89/01930
- WO-A-94/28883
- WO-A-95/12572
- WO-A-95/21815
- WO-A-98/52549
- WO-A-99/21565
- WO-A-99/49858
- WO-A-02/055063
- WO-A-02/055066
- DE-A- 2 212 369
- DE-A- 2 417 788
- DE-A- 2 530 372
- DE-A- 2 543 351
- DE-A- 2 621 214
- DE-A- 3 834 794
- DE-A- 10 000 577
- DE-A- 10 101 307
- DE-A- 19 721 099
- DE-A- 19 839 566
- DE-A- 19 853 487
- FR-A- 1 563 486
- US-A- 3 139 395
- US-A- 3 253 016
- US-A- 3 920 837
- US-A- 4 086 334
- US-A- 4 959 389
- US-A- 5 042 986
- US-A- 5 424 332
- GRIFFIN GW, VELLTURO AF, FURUKAWA K: "The Chemistry of Photodimers of Maleic and Fumaric Acid Derivatives. I. Dimethyl Fumarate Dimer" JOURNAL OF THE AMERICAN CHEMICAL SOCIETY, Bd. 83, 1961, Seiten 2725-2728, XP002245338
- FARINA M, GRASSI M, DI SILVESTRO G: "Stereochemical Study of 1,2,3,4,5,6-Hexakis(methoxycarbonyl)cycloh exanes" JOURNAL OF THE AMERICAN CHEMICAL SOCIETY, Bd. 107, 1985, Seiten 5100-5104, XP002245339
- SEBÖK B ET AL: "Effect of Fumaric Acid, Its Dimethylester, and Topical Antipsoriatic Drugs on Epidermal Differentiation in the Mouse Tail Model" SKIN PHARMACOLOGY, S. KARGER, BASEL, CH, Bd. 9, Nr. 2, 1. März 1996 (1996-03-01), Seiten 99-103, XP002088946 ISSN: 1011-0283
- BAYARD W ET AL: "Perorale Langzeitbehandlung der Psoriasis mit Fumarsäurederivaten" HAUTARZT, SPRINGER VERLAG, BERLIN, DE, Bd. 5, Nr. 38, 1987, Seiten 279-285, XP002076555 ISSN: 0017-8470
- ALTMEYER P ET AL: "[Systemic therapy of psoriasis ]. SYSTEMISCHE THERAPIE DER PSORIASIS" T&E DERMATOLOGIE, BRAUN, KARLSRUHE, DE, Bd. 27, Nr. 6, 1997, Seiten 380-382,384, XP002107172 ISSN: 0939-0448
- HUNZIKER T ET AL: "Psoriasis, eine Autoimmunkrankheit?" THERAPEUTISCHE UMSCHAU, VERLAG HANS HUBER, BERN, CH, Bd. 50, Nr. 2, 1993, Seiten 110-113, XP002088941 ISSN: 0040-5930
- MAIER, M.E.: "Synthesis of Medium-Sized Rings by the Ring-Closing Metathesis Reaction" ANGEW. CHEM. IND. ED., Bd. 39, Nr. 12, 2000, Seiten 2073-2077, XP002257238

## Beschreibung

Die vorliegende Erfindung betrifft bestimmte carbocyclische Fumarsäure-Oligomere sowie die Verwendung derselben zur Herstellung eines Arzneimittels und diese Verbindungen enthaltende Arzneimittel.

Fumarsäuredialkylester sowie Fumarsäuremonöalkylester und Salze derselben werden seit langem mit Erfolg zur Behandlung der Psoriasis verwendet. Diese Verwendung ist in einer Anzahl von Patenten beschrieben worden, siehe beispielsweise die DE-25 30 372, DE 26 21 214 oder EP-B-0 312 697.

Weiterhin beschrieben ist die Verwendung der Fumarsäuremono- und -diester zur Behandlung von Autoimmunerkrankungen, wie beispielsweise der Polyarthritis oder der Multiplen Sklerose (siehe DE 197 21 099.6 sowie DE 198 53 487.6), aber auch zur Verwendung in der Transplantationsmedizin (siehe DE 198 53 487.6 und DE 198 39 566.3). Aus der DE 101 01 307.8 sowie der DE 100 00 577.2 ist außerdem die Verwendung der Fumarsäuremono- und -diester zur Behandlung NFkappaB vermittelter Erkrankungen wie die Behandlung mitochondrialer Erkrankungen bekannt. Alle genannten Druckschriften beschreiben Fumarsäuremono- und -diester, gegebenenfalls in Form bestimmter Salze.

Die genannten Fumarsäureester weisen wegen deren Flüchtigkeit bzw. Sublimierbarkeit den Nachteil auf, dass sie bei der Herstellung pharmazeutischer Produkte, insbesondere in fester Form zur oralen Verabreichung, schwierig zu handhaben sind. Genauer erfordert die Herstellung dieser Produkte Schutzmaßnahmen wie die Verwendung von Atemmasken, Handschuhen, Schutzanzügen usw.

Darüber hinaus werden die Fumarsäureester nach oraler Verabreichung im Magen-Darm-Trakt resorbiert und von allen Körperzellen unspezifisch aus dem Blutstrom aufgenommen. Es müssen daher entsprechend hohe Dosen verabreicht werden. Diese hohen Dosen führen wiederum zu den bekannten Nebenwirkungen der Fumarsäuretherapie wie Flush-Symptome (Rötungen) oder gastrointestinalen Reizungen (Übelkeit, Durchfälle, Blähungen). Die Nebenwirkungen können zwar, wie im oben genannten Stand der Technik beschrieben, durch die Verabreichung des Wirkstoffes in Form von Mikrotabletten erheblich gesenkt, jedoch nicht vollständig vermieden werden.

Gleichzeitig werden die Fumarsäureester im Blut rasch hydrolysiert und die Hydrolyseprodukte Alkohol und Fumarsäure bzw. Fumarsäuremonoester verstoffwechselt. Zum Erhalt therapeutisch wirksamer Spiegel ist daher eine wiederholte, häufig Verabreichung erforderlich. Zwar lässt sich bezüglich der Nebenwirkungen ein gewisser Gewöhnungseffekt beobachten, eine weitere Verringerung der Nebenwirkungsrate wäre jedoch wünschenswert.

Zur Vermeidung dieser Nachteile sind aus der nicht vorveröffentlichten DE 101 33 004.9 sind Fumarsäuremono- und -diamide bekannt. Diese Amide werden mit Aminosäuren und vorzugsweise mit bestimmten Peptiden gebildet. Die Bindung an ein Peptid soll für die spezifische Übermittlung des Fumarsäurederivats an einzelne Zielzellen sorgen. Die genannten Fumarsäure-Peptid-Derivate weisen jedoch den Nachteil auf, dass sie in der Herstellung sehr aufwendig sind.

Es ist daher Aufgabe der vorliegenden Erfindung, Fumarsäurederivate und deren Verwendung zur Verfügung zu stellen, die beständiger gegenüber der Hydrolyse und einfacher in Herstellung und Handhabung sind.

Gelöst wird die vorliegende Aufgabe durch bestimmte carbocyclische Fumarsäure-Oligomere zur Verwendung als Arzneimitteln, deren Verwendung zur Herstellung von Arzneimitteln und diese enthaltende Arzneimittel formulierungen.

Genauer betrifft die vorliegende Erfindung in einem ersten Aspekt carbocyclische Fumarsäure-Oligomere, zur Verwendung als Arzneimittel wie in den Diese anliegenden Ansprüchen 1 und 2 definiert. carbocyclischen Fumarsäure-Oligomere werden vorzugsweise durch olefinischeder C-C-Doppelbindungen bzw.polarisierte olefinische Polymerisation der C-C-Doppelbindungen und Carbonylsauerstoffe der Einheiten erhalten. Vorzugsweise sind die von der Fumarsäure abgeleiteten Einheiten abgeleitet von Monomeren, ausgewählt aus der Gruppe, bestehend aus Fumarsäure, Dialkylfumaraten, Monoalkylhydrogenfumaraten, und deren Salzen sowie Mischungen derselben. Stärker bevorzugt enthält das erfindungsgemäße Oligomer lediglich von einem oder zwei Monomeren abgeleitete Einheiten, am meisten bevorzugt enthält das Oligomer ausschließlich identische Monomereinheiten. Bevorzugt handelt es sich bei dem Monomeren nicht um die Fumarsäure selbst, sondern um eines der genannten Derivate, insbesondere die Mono- oder Dialkylfumarate.

Das erfindungsgemäße carbocyclische Oligomer setzt sich aus den von der Fumarsäure abgeleiteten Einheiten dergestalt zusammen bzw. besteht aus diesen, dass die Einheiten an den C-Atomen 2 und 3 des Fumarsäureskeletts durch kovalente C-C-Bindungen so miteinander verbunden sind, dass ein carbocyclisches Oligomer entsteht. Die C-C-Bindungen können durch olefinische Polymerisation der Doppelbindungen entstehen. Das erfindungsgemäße carbocyclische Fumarsäure-Oligomer enthält keine olefinischen Unsättigungen im Rückgrat.

Das Oligomerrückgrat (der Carbocyclus) besteht bei dem erfindungsgemäßen carbocyclischen Oligomeren aus den Fumarsäure-Einheiten, d.h. es weist eine gerade Anzahl an C-Atomen auf und enthält keine anderen Monomeren und/oder Heteroatome. Dieses Rückgrat ist an jedem C-Atom durch eine der Carbonsäure- gruppen der Fumarsäure-Monomereinheit(en) substituiert, aus der bzw. denen es aufgebaut ist. Die Monomereinheiten können bei der Synthese bzw. über Polymerisation der Derivate in Form der Ester, aber auch in Form von Salzen vorliegen.

Der hier verwendete Ausdruck "Oligomer" bezieht sich auf eine Anzahl von mindestens zwei Fumarsäure-Monomereinheiten. Das erfindungsgemäße carbocyclische Fumarsäure-Oligomer enthält 2 2 von Fumarsäure abgeleitete Einheiten. Die Carbonsäure- bzw. Carbonsäureamidgruppen als Substituenten der Fumarsäure-Einheiten stehen stehen im erfindungsgemäßen Oligomer alle in trans-Stellung zueinander, d.h. zu beiden jeweils benachbarten Carbonsäure- bzw. Cabonsäureamidgruppen.

In einer bevorzugten Ausführungsform betrifft die Erfindung ein carbocyclisches Fumarsäure-Oligomer, entsprechend der folgenden Formel (II): 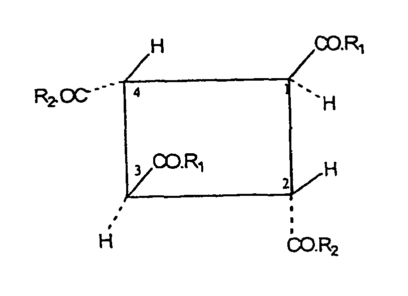 worin die Reste R1 und R2 gleich oder verschieden sind und ausgewählt sind aus des Gruppe, bestehend aus Alkoholresten (-OR5) und einem Hydroxylrest, der Rest R5 ausgewählt ist aus der Gruppe, bestehend aus Wasserstoff und C₁₋₂₄-Alkylresten.

In einer ersten Ausführungsform stellen die Reste R1 und R2 bevorzugt unabhängig je einen Alkohol- oder Hydroxylrest dar. Vorzugsweise bedeuten R1 und R2 nicht gleichzeitig Hydroxyl. Bei dem oder den Monomeren handelt es sich vorzugsweise also um ein oder mehrere Monoalkylhydrogenfumarat(e). In einer anderen Ausführungsform können beide Reste R1 und R2 einen Alkoxyrest -OR5 darstellen, der noch bevorzugter identisch ist. In diesem Fall handelt es sich bei dem oder den Monomeren um Dialkylfumarate.

Bevorzugt sind R1 und R2 unabhängig voneinander ausgewählt aus der Gruppe, bestehend aus Hydroxyl, Methoxy, Ethoxy, Propoxy, Isopropoxy, n-Butoxy, sec.-Butoxy, tert.-Butoxy, Pentoxy, bevorzugt Methoxy und/oder Ethoxy. Ganz besonders bevorzugt sind demgemäß carbocyclische Oligomere die sich von Dimethylfumarat, Diethylfumarat, Methylethylfümarat, Methylhydrogenfumarat und Ethylhydrogenfumarat ableiten. Am meisten bevorzugt ist ein carbocyclisches Fumarsäure-Oligomer der Formel (I), worin R1 und R2 beide identisch Methoxy oder Ethoxy bedeuten.

Eine Carboxyfunktion tragende Monomere und die entsprechenden erfindungsgemäßen Poly-mere (in denen R1 und/oder R2 = -OH bzw. -O bedeuten) können selbstverständlich in Form ihrer Salze vorliegen. Bevorzugt sind die Alkalimetallsalze wie Li, Na, K, die Erdalkalimetallsalze wie Mg, Ca sowie die Salze physiologisch annehmbarer Übergangsmetalle, insbesondere Fe und Zn.

Bevorzugt sind die Reste R5 gleich oder verschieden und sind ausgewählt aus der Gruppe, bestehend aus Wasserstoff, Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl, 2-Hydroxyethyl, 2- oder 3-Hydroxypropyl, 2,3-Dihydroxypropyl, 2-Methoxyethyl, Methoxymethyl und 2- oder 3-Methoxypropyl.

In einer ganz besonders bevorzugten Ausführungsform betrifft die Erfindung ein carbocyclisches Fumarsäure-Oligomer, entsprechend der Formel (II): 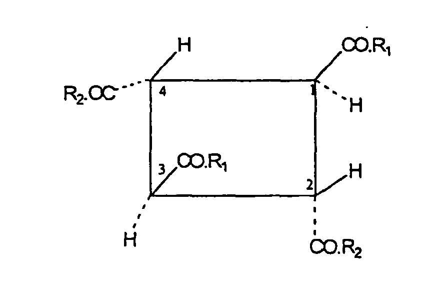 das als r-1,t-2,c-3,t-4-Tetrakis(alkoxycarbonyl)cyclobutan oder r-1,t-2,c-3,t-4-Cyclobutantetracarbonsäurealkylester bezeichnet werden kann.

Am meisten bevorzugt betrifft die Erfindung ein carbocyclisches Fumarsäure-Oligomer, entsprechend der Formel (IIa): 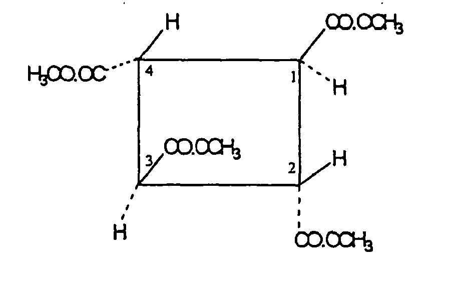 das als r-1,t-2,c-3,t-4-Tetrakis(methoxycarbonyl)cyclobutan oder r-1,t-2,c-3,t-4-Cyclobutantetracarbonsäuremetlylester bezeichnet werden kann.

Die erfindungsgemäßen carbocyclischen Fumarsäure-Oligomere können über allgemein bekannte Verfahren zur Herstellung zyklischer Verbindungen hergestellt werden. Die Herstellung kann beispielsweise mit Hilfe bekannter Zyklisierungsmittel wie Borverbindungen, Polyphosphorsäuren etc. unter üblichen Bedingungen erfolgen.

Bevorzugt werden die erfindungsgemäßen carbocyclischen Fumarsäure-Oligomere über ein Photopolymerisationsverfahren hergestellt. Wie bei diesen Verfahren üblich wird die Polymerisation durch Bestrahlung der Monomere, meist in flüssiger Phase ggf. in Kombination mit einem hierfür geeigneten, üblichen Lösungsmittel, das gegenüber der Polymerisation inert ist wie einem Alkan, Cycloalkan oder aromatischen Lösungsmittel, mit Licht einer Wellenlänge von 200 bis 700 nm induziert. Falls gewünscht können übliche Polymerisationsinitiatoren wie Hydroperoxide, organische Peroxide, Benzoinmethylether, Benzyl oder Diacetyl etc. und/oder Sensibilisierungsmittel zugesetzt werden, bspw. um die Ausbeute der Reaktion zu erhöhen. Zur Aktivierung der ethylenischen Unsättigungen der Fumarsäure-Monomereinheiten bzw. anderer entsprechender Monomereinheiten werden vorzugsweise Wellenlängen im UV- oder Blaulichtbereich verwendet.

Eine weitere bevorzugte Herstellungsmethode ist die sogenannte Metathese, die heute das Mittel der Wahl für gezielte Polymerisationen oder Ringschluss-Synthesen darstellt. Bei den als Metathesereaktionen bezeichneten Reaktionen handelt es sich im allgemeinen um von Schwermetallen katalysierte Zyklisierungen oder Polymerisationen. Einen allgemeinen Überblick gibt der Artikel "Die Olefinmetathese - neue Katalysatoren vergrößern das Anwendungspotential" von M. Schuster und S. Blechert, Chemie in unserer Zeit, Nr.1, 2001.

Die Metathesereaktionen zur Herstellung der erfindungsgemäßen Fumarsäure-Oligomere können unter Anwendung der üblichen Bedingungen und der üblichen Katalysatoren als homogene oder heterogene Reaktionen durchgeführt werden. Für die Katalysatoren sind beispielhaft die Katalysatoren auf Basis von Pd, Mo und Ru zu nennen, insbesondere der Grubb'sche Katalysator und der Schrock'sche Katalysator. Die Metathesen können in herkömmlichen Lösungsmitteln wie ggf. halogenierten Kohlenwasserstoffen, insbesondere Alkanen, Cycloalkanen, aromatischen Lösungsmitteln, aber auch Ethern, Estern, DMSO usw. durchgeführt werden. Die Reaktionstemperaturen liegen üblicherweise unterhalb von Raumtemperatur, beispielsweise zwischen -20 und 10 °C.

Die Herstellung der erfindungsgemäßen carbocyclischen Fumarsäure-Oligomere kann auch über Kombinationen der genannten Verfahren, beispielsweise zunächst über die Photopolymerisation zum Erhalt von cyclischen und/oder linearen Polymeren und anschließende Ringschlussmetathese, ggf. in Form der Abspaltung des zyklisierten Moleküls erfolgen (vgl. J. Pernerstorfer, M. Schuster und S. Blechert in "Cyclisation/cleavage of macrocycles by ring closing metathesis on solid support - confirmational studies", Chem. Commun. 1997, 1949).

In einem zweiten Aspekt betrifft die Erfindung die Verwendung eines wie oben definierten carbocyclischen Fumarsäure-Oligomeren zur Herstellung eines Arzneimittels gemäß Anspruch 6. In einem dritten Aspekt betrifft die vorliegenden Erfindung außerdem eine Arzneimittelformulierung gemäß Anspruch 7, enthaltend ein wie oben definiertes Fumarsäure-Oligomer.

Vorzugsweise ist das Arzneimittel zur Behandlung einer Autoimmunerkrankung, in der Transplantationsmedizin, zur Behandlung von mitochondrialen Erkrankungen und von NFkappaB beeinflußbaren Erkrankungen bestimmt. Das Arzneimittel ist vorzugsweise insbesondere bestimmt und geeignet
(1) zur Therapie einer Autoimmunerkrankung ausgewählt aus der Gruppe bestehend aus der Polyarthritis, Multiplen Sklerose, Graft-versus-Host-Reaktionen, dem juvenilen Diabetes, der Hashimoto-Tyreoiditis, der Grave's disease (Graves Krankheit oder Basedow Krankheit), dem systemischen Lupus erythematodes (SLE), dem Sjögren Syndrom (Sjogren's Syndrome), der perniziösen Anämie und der chronischen aktiven (= lupoiden) Hepatitis,
(2) zur Verwendung in der Transplantationsmedizin (Host-versus-Graft-Reaktionen),
(3) zur Therapie mitochondrialer Erkrankungen, ausgewählt aus der Gruppe bestehend aus dem Parkinson-Syndrom, der Alzheimer-Krankheit, der Chorea-Huntington-Krankheit, der Retinopathia pigmentosa oder mitochondrialen Enzephalomyopathien, sowie
(4) zur Therapie von NFkappaB vermittelten Erkrankungen, ausgewählt aus der Gruppe, bestehend aus der progressiven systemischen Sklerodermie, der Osteochondritis syphilitica (Wegener's Disease), der Cutis marmorata (Livedo Reticularis), der Behcet-Disease, Panarteritis, Colitis ulcerosa, Vasculitis, der Osteoarthritis, Gicht, Ateriosklerosis, der Reiter's Erkrankung, der bronchozentischen Granulomatose, Encephalitis-Typen, dem Endotoxin-Schock (septisch-toxischer Schock), der Sepsis, der Pneumonie, der Encephalomyelitis, der Anorexia nervosa, der Hepatitis (der akuten Hepatitis, der chronischen Hepatitis, der toxischen Hepatitis, der Alkoholhepatitis, der viralen Hepatitis, der Gelbsucht, der Leberinsuffizienz und der cytomegaloviralen Hepatitis), der Rennert T Lymphomatosis, der mesangialen Nephritis, der Postangioplastie-Restenose, das Reperfusionssyndrom, der cytomegaloviralen Retinopathie, Adenoviralen Erkrankungen wie adenoviralen Erkältungserkrankungen, dem adenoviralen Pharyngoconjunctivalfieber, und der adenoviralen Ophthalmie, AIDS, dem Guillain-Barré-Syndrom, der postherpetischen oder postzoster Neuralgie, der inflammatorischen demyelinisierenden Polyneuropathie, der Mononeuropathia multiplex, der Mukoviszidose, Morbus Bechterew, Barett-Ösophagus, EBV-(Epstein-Barr-Virus)-Infektion, dem kardialen Remodeling, interstitiellen Zystitis, Diabetes mellitus Typ II, der Strahlensensibilisierung maligner Tumore, der Mehrfachresistenz maligner Zellen auf Chemotherapeutika, Granuloma annulare und Krebserkrankungen wie Mamma Karzinom, Kolonkarzinom, Melanom, primäres Leberzellkarzinom, Adenokarzinom, Kaposi Sarkom, Prostatakarzinom, Leukämie wie der akuten myeloischen Leukämie, dem multiplen Myelom (Plasmozytom), Burkitt-Lymphom, und dem Castleman-Tumor.

Das Arzneimittel kann in für die orale, rektale, transdermale, ophtalmologische, nasale, pulmonale oder parenterale Applikation geeigneter Form vorliegen. Vorzugsweise ist das Arzneimittel für die orale Verabreichung geeignet. Es kann dann in Form von Tabletten, Dragees, Kapseln, Granulat, Trinklösungen, Liposomen, Nanopartikeln, Nanokapseln, Mikrokapseln, Mikrotabletten, Pellets oder Pulvern sowie in Kapseln gefülltem Granulat, in Kapseln gefüllten Mikrotabletten, in Kapseln gefüllten Pellets, in Kapseln gefüllten Nanopartikeln oder in Kapseln gefülltem Pulver vorliegen. Vorzugsweise liegt das Arzneimittel in Form von Nanopartikeln, Milcropellets oder Mikrotabletten vor, die ggf. in Sachets oder Kapseln abgefüllt sein können. Diese Mikropellets oder Mikrotabletten weisen üblicherweise einen Durchmesser, ohne Beschichtung, von ≤ 5000 µm auf, vorzugsweise 300 bis 2000 µm.

Bevorzugt können alle festen oralen Dosisformen mit einer magensaftresistenten Beschichtung versehen sein. Diese kann beispielsweise auf den Tabletten, Mikrotabletten Mikropellets etc. aufgebracht sein, kann aber auch auf diese enthaltenen Kapseln aufgetragen werden.

Die erfindungsgemäßen Arzneimittelformen können grundsätzlich nach der klassischen Tablettiermethode aber auch über Direkttablettierung sowie feste Dispersionen nach der Schmelzmethode und über die Sprühtrocknungsmethode hergestellt werden. Ein magensaftresistenter Überzug kann, falls gewünscht, nach bekannten Verfahren in einem klassischen Dragierkessel aufgeleert oder aufgesprüht sowie in einer Wirbelschichtapparatur aufgetragen werden. Nach beendeter Trocknung kann anschliessend in der gleichen Apparatur ein Filmcoat aufgebracht werden. Bei Verwendung von Wirkstoffgemischen können auch Pellets mit den einzelnen Wirkstoffen hergestellt und diese, ggf. nach Befilmung, in den gewünschten Mengen gemischt werden.

Bei parenteraler Verabreichung liegt das Arzneimittel in hierfür geeigneter Form bspw. als sterile Lösung oder Emulsion vor. Entsprechende Formulierungen und hierfür geeignete Hilfsmittel sind dem Fachmann bekannt.

Das erfindungsgemäße Arzneimittel enthält eine für den therapeutischen Zweck angemessene Menge an Oligomer. Diese kann von Fachmann anhand von Routineversuchen ermittelt werden. Üblicherweise wird das Arzneimittel eine Menge an Fumarsäure-Oligomer enthalten, die 10 bis 500 mg Fumarsäure, bevorzugt 30 bis 200 mg Fumarsäure und am meisten bevorzugt 100 mg Fumarsäure entspricht.

Die Verwendung der erfindungsgemäßen Oligomere bietet gegenüber der bekannten Verwendung der Monomere als Arzneimittelwirkstoffe den Vorteil, dass sie aufgrund des höheren Molekulargewichts weniger flüchtig sind und daher ein der Herstellung und Verarbeitung leichter zu handhaben sind. Sie bieten darüber hinaus den Vorteil, dass sie als synthetische Substanzen im Körper zunächst in körpereigene Substanzen überführt werden müssen, was ihre Verweilzeit in Organismus erhöht. Dies erfolgt vermutlich durch Aufspaltung in die Monomeren. Aufgrund der Oligomerisation weisen sie außerdem den Vorteil auf, dass sie weniger reizend auf die Schleimhäute wirken und damit weniger Nebenwirkungen aufweisen.

Die Erfindung soll nun anhand der folgenden Beispiele veranschaulicht werden, die diese jedoch nicht beschränken sollen.

### Beispiele

### Beispiel 1

### Herstellung von magensaftresistenten Mikrotabletten in Kapseln, enthaltend 60,0 mg r-1,t-2,c-3,t-4-Tetrakis(methoxycarbonyl)cyclobutan und 30.0 mg r-1,t-2,c-3,t-4,c-5, t-6 - Hexa(methoxycarbonyl)cyclohexan

6,0 kg r-1 ,t-2 ,c-3 ,t-4-Tetrakis(methoxycarbonyl)cyclobutan und 3,0 kg r-1,t-2,c-3-,t-4, c-5, t-6 - Hexa(methoxycarbonyl)cyclohexan werden zerkleinert, intensiv gemischt und mittels eines Siebs 800 homogenisiert. Es wird ein Hilfsstoffgemisch folgender Zusammensetzung hergestellt: 18,00 kg Stärkederivat (STA-RX 1500), 0,30 kg Cellulose mikrokristallin (Avicel PH 101), 0,75 kg PVP (Kollidon 120), 4,00 kg Primogel, 0,25 kg Kieselsäure kolloidal (Aerosil). Das gesamte Pulvergemisch wird mit dem Wirkstoffgemisch versetzt und mittels eines Siebs 200 homogenisiert und mit einer 2%igen wässrigen Lösung von Polyvinylpyrrolidon (Kollidon K25) auf übliche Weise zu einem Bindemittelgranulat verarbeitet und in trockenem Zustand mit der äußeren Phase gemischt. Diese besteht aus 0,50 kg Mg-Stearat und 1,50 kg Talkum. Das Pulvergemisch wird anschliessend auf üblicher Weise zu gewölbten Mikrotabletten von 10,0 mg Bruttomasse und 2,0 mm Durchmesser gepresst.

Zum Erreichen der Magensaftresistenz wird portionsweise eine Lösung von 2,250 kg Hydroxypropylmethylcellulosephthalat (HPMCCP, Pharmacoat HP 50), in einem Gemisch folgender Lösungsmittel aufgelöst: Aceton 13,00 1, Ethanol 94 Gewichtsprozent denaturiert mit 2% Keton 13,50 1 und Aqua demineralisata 2,50 1. Zu der fertigen Lösung wird als Weichmacher Rizinusöl 0,240 kg zugegeben und auf übliche Weise in Portionen auf die Tablettenkerne aufgetragen.

Nach beendeter Trocknung wird anschliessend in der gleichen Apparatur eine Suspension folgender Zusammensetzung als Filmcoat aufgetragen: Talk 0,340 kg, Titan (VI)-oxyd Cronus RN 56 0,400 kg, Farblack L-Rotlack 86837 0,324 kg, Eudragit E 12,5% 4,800 kg und Polyethylenglycol 6000 pH 11 XI 0,120 kg in einem Lösungsgemisch folgender Zusammensetzung: 2-Propanol 8,170 kg, Aqua demineralisata 0,200 kg und Glycerintriacetat (Triacetin) 0,600 kg.

Die magensaftresistenten Mikrotabletten werden anschliessend auf den Wirkstoffgehalt analysiert, in Hartgelatine-Steckkapseln zum entsprecheden netto Gewicht eingefüllt und verschlossen.

### Beispiel 2

### Herstellung von magensaftresistenten Mikrotabletten in Kapseln, enthaltend 60,0 mg r-1,t-2,c-3,t-4-Tetrakis(methoxycarbonyl)cyclobutan und 30.0 mg r-1,t-2,c-3,t-4, c-5, t-6- Hexa(metboxycarbonyl)cyclohexan

6,0 kg r-1,t-2,c-3,t-4-Tetrakis(methoxycarbonyl)cyclobutan und 3,0 kg r-1,t-2,c-3,t-4, c-5, t-6 - Hexa(methoxycarbonyl)cyclohexan werden zerkleinert, intensiv gemischt und mittels eines Siebs 800 homogenisiert. Es wird ein Hilfsstoffgemisch folgender Zusammensetzung hergestellt: 24,70 kg mikrokristalline Cellulose (Avicel PH 200), 3,00 kg Croscarmellose Natrium (AC-Di-SOL-SD-711), 2,50 kg Talkum, 0,10 kg Siliciumdioxid wasserfrei (Aerosil 200) und 1,00 kg Magnesiumstearat. Das gesamte Hilfsstoffgemisch wird mit dem Wirkstoffgemisch versetzt und homogen gemischt. Das Pulvergemisch wird anschliessend mittels Direkttablettierung zu gewölbten Mikrotabletten von 10,0 mg Bruttomasse und 2,0 mm Durchmesser gepresst.

Als magensaftresistenter Ueberzug wird eine Lösung von 0,94 kg Eudragit L in Isopropanol hergestellt, die zusätzlich 0,07 kg Dibutylphthalat enthält. Diese Lösung wird auf die Tablettenkerne aufgesprüht. Danach wird eine Dispersion von 17,32 kg Eudragit L D-55 und einer Mischung aus 2,80 kg Mikrotalkum, 2,00 kg Macrogol 6000 und 0,07 kg Dimeticon in Wasser hergestellt und auf die Kerne aufgesprüht.

Die magensaftresistenten Mikrotabletten werden anschließend auf den Wirkstoffgehalt analysiert, in Hartgelatine-Steckkapseln oder in Beutel zum entsprechenden netto Gewicht eingefüllt und verschlossen.

### Beispiel 3

### Herstellung von magensaftresistenten Pellets in Kapseln, enthaltend 60,0 mg r-1,t-2, c-3,t-4-Tetrakis(methoxycarbonyl)cyclobutan und 30.0 mg r-1,t-2,c-3,t-4, c-5, t-6 - Hexa(methoxycarbonyl)cyclohexan

6,0 kg r-1,t-2,c-3,t-4-Tetrakis(methoxycarbonyl)cyclobutan und 3,0 kg r-1,t-2,c-3,t-4, c-5, t-6 - Hexa(methoxycarbonyl)cyclohexan werden zerkleinert, intensiv gemischt und mittels eines Siebs 800 homogenisiert. Daneben wird 2 1 einer 20% (m/V) Polyvinylpyrrolidon (Kollidon K30) Lösung in Ethanol vorbereitet. 7,250 kg Nonpareilles Pellets werden in einem Dragierkessel belegt und mit einem Teil der Kollidon K-30 Lösung besprüht bis diese leicht feucht werden. Das Wirkstoffgemisch wird danach portionsweise zugegeben bis zum Auftrocknen der Pellets. Diese Vorgehensweise der Befeuchtung/Auftrocknung wird bis zur endgültigen Zugabe des Wirkstoffgemisches weitergeführt. Der Rest der PVP Lösung wird mit 0,720 kg Eudragit E 12.5% Lösung gemischt und ganz auf die Pellets gesprüht. Die Pellets werden bis zum vollständigen Austrocknen bewegt.

Die Pellets werden mit Eudragit S 12.5% Lösung besprüht und mit Talk aufgetrocknet. Nach jedem Besprühungs-/Auftrocknungszyklus wird die Freisetzung des Wirkstoffes gemessen und Eudragit S 12.5% Lösung/Talkum weiter zugegeben bis eine Freisetzung gemäss Spezifikation erhalten wird.

Die magensaftresistenten Pellets werden danach auf den Wirkstoffgehalt analysiert, in Kapseln abgefüllt (auf das entsprechende netto Gewicht pro Kapsel).

### Beispiel 4

### Herstellung von magensaftresistenten Tabletten, enthaltend 120,0 mg r-1,t-2,c-3,t-4-Tetrakis(methoxycarbonyl)cyclobutan

12,0 kg r-1,t-2,c-3,t-4-Tetrakis(methoxycarbonyl)cyclobutan werden zerkleinert, intensiv gemischt und mittels eines Siebes 800 homogenisiert. Anschliessend wird ein Hilfsstoffgemisch folgender Zusammensetzung hergestellt: 19,000 kg Stärkederivat (STA-RX 1500^{®}), 2,000 kg mikrokristalline Cellulose (Avicel PH 101^{®}), 0,600 kg Polyvinylpyrolidon (PVP, Kollidon^{®}25), 4,000 kg Primogel^{®}, 0,300 kg kolloidaler Kieselsäure (Aerosil^{®}). Das gesamte Pulvergemisch wird mit dem Wirkstoff versetzt, gemischt, mittels eines Siebes 200 homogenisiert und mit einer 2 % igen wässrigen Lösung von Polyvinylpyrolidon (PVP, Kollidon^{®}25) auf übliche Weise zu einem Bindemittelgranulat verarbeitet - und in trockenem Zustand mit der äusseren Phase gemischt. Diese besteht aus 2,000 kg eines sogenannten FST-Komplexes, enthaltend 80% Talk, 10% Kieselsäure und 10% Magnesiumstearat. Es wird anschliessend auf übliche Weise zu gewölbten Tabletten von 400 mg Gewicht und 11,5 mm Durchmesser gepresst.

Es wird eine Lösung von 2,250 kg Hydroxypropylmethylcellulosephthalat (HPMCP, Pharmacoat HP^{®}50) in einem Lösungsmittelgemisch von 2,50 1 demineralisiertem Wasser, 13,00 1 Aceton Ph.Helv.VII und 13,00 1 Ethanol 94 Gewichtsprozent gelöst und die Lösung mit 0,240 kg Rizinusöl (Ph.Eur. II) versetzt. Die Lösung wird im Dragierkessel auf traditionelle Weise in Portionen auf die Tablettenkerne aufgeleert oder aufgesprüht.

Nach entsprechender Trocknung wird anschliessend ein Filmüberzug angebracht. Dieser setzt sich zusammen aus einer Lösung von Eudragit E 12,5%^{®} 4,800 kg, Talcum Ph. Eur. II 0,340 kg, Titan (VI)-oxyd Cronus RN 56^{®} 0,520 kg, Farblack ZLT-2 blau (Siegle) 0,210 kg, und Polyethylenglycol 6000 Ph.Helv.VII 0,120 kg in einem Lösungsmittelgemisch von 8,200 kg 2-Propanol Ph.Helv.VII, 0,060 kg Glycerintriacetat (Triacetin^{®}) und 0,200 kg Aqua demineralisata. Nach homogener Verteilung im Wirbelschichtbett wird getrocknet und auf übliche Weise poliert.

### Beispiel 5

### Herstellung einer Suspension zur parenteralen Applikation, enthaltend 60,0 mg r-1, t-2,c-3,t-4-Tetrakis(methoxycarbonyl)cyclobutan und 30.0 mg r-1,t-2,c-3,t-4, c-5,t-6 Hexa(methoxycarbonyl)cyclohexan

| Inhaltsstoffe | mg/ml |
|---|---|
| r-1,t-2,c-3,t-4-Tetrakis(methoxycarbonyl)cyclobutan | 60.00 |
| r-1,t-2,c-3,t-4, c-5, t-6 - Hexa(methoxycarbonyl)cyclohexan | 30.00 |
| Methylcellulose | 0.25 |
| Natriumcitrat, Dihydrat | 30.00 |
| Benzylalkohol | 9.00 |
| Methylparaben | 1.80 |
| Propylparaben | 1.20 |
| Wasser für Injektionszwecke | q.s.a.d. 1.00 |

Unter Verwendung von Standard-Techniken werden die oben genannten Inhaltsstoffe zu einer parenteralen Suspension verarbeitet.

### Beispiel 6

### Herstellung einer Lösung zur parenteralen Applikation, enthaltend 30,0 mg r-1,t-2, c-3,t-4-Tetrakis (methoxycarbonyl)cyclobutan

| Inhaltsstoffe | mg/ml |
|---|---|
| r-1,t-2,c-3,t-4-Tetrakis(methoxycarbonyl)cyclobutan | 30.00 |
| Hydroxypropyl-beta-cyclodextrin | 300.00 |
| Natriumdihydrogenphosphat | 10.00 |
| Methylparaben | 0.75 |
| Monothioglycerol | 2.00 |
| Wasser für Injektionszwecke | q.s.a.d. 1.00 |

Unter Verwendung von Standard-Techniken werden die oben genannten Inhaltsstoffe zu einer parenteralen Lösung verarbeitet.

## Patentansprüche

1. Carbocyclisches Oligomer zur Verwendung als Arzneimittel, das carbocyclische Oligomer bestehend aus 2 von Fumarsäure oder deren Estern abgeleiteten Einheiten als wiederkehrende Einheiten, erhältlich durch Polymerisation der C-C-Doppelbindungen der Einheiten und entsprechend der folgenden Formel (II): 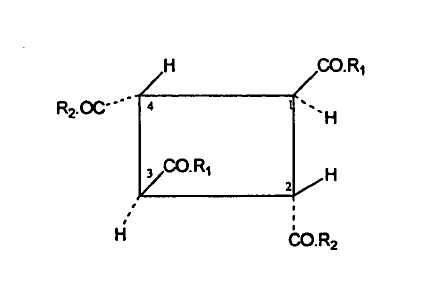 worin die Reste R1 und R2 gleich oder verschieden sind und ausgewählt sind aus der Gruppe, bestehend aus Alkoholresten (-OR⁵) und der Rest R⁵ ausgewählt ist unter Wasserstoff und C₁₋₂₄-Alkylresten.

2. Carbocyclisches Oligomer zur Verwendung als Arzneimittel, das carbocyclische Oligomer bestehend aus 2 von Fumarsäure oder deren Estern abgeleiteten Einheiten als wiederkehrende Einheiten, erhältlich durch Polymerisation der C-C-Doppelbindungen der Einheiten und entsprechend der folgenden Formel (II): 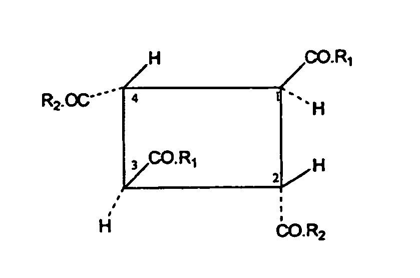 worin die Reste R1 und R2 gleich oder verschieden sind und ausgewählt sind aus der Gruppe, bestehend aus Alkoholresten (-OR⁵) und
der Rest R⁵ ausgewählt aus der Gruppe, bestehend aus Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl, 2-Hydroxyethyl, 2- oder 3-Hydroxypropyl, 2,3-Dihydroxypropyl, 2-Methoxyethyl, Methoxymethyl und 2- oder 3-Methoxypropyl.

3. Oligomer nach Anspruch 1, worin R1 und R2 unabhängig je einen Alkoxy- oder Hydroxylrest darstellen, aber nicht beide -OH sein können.

4. Oligomer nach Anspruch 3, worin R1 und R2 unabhängig voneinander ausgewählt sind aus der Gruppe, bestehend aus Methoxy, Ethoxy, Propoxy, Isopropoxy, n-Butoxy, sec.-Butoxy, tert.-Butoxy und Pentoxy.

5. Oligomer nach Anspruch 3, worin R1 und R2 beide Methoxy bedeuten.

6. Verwendung eines carbocyclischen Oligomeren nach einem der vorstehenden Ansprüche zur Herstellung eines Arzneimittels, worin das Arzneimittel bestimmt ist zur Behandlung einer Autoimmunerkrankung, in der Transplantationsmedizin, zur Behandlung von mitochondrialen Erkrankungen und von NFkappaB beeinflußbaren Erkrankungen.

7. Arzneirnittelformulierung, enthaltend ein Fumarsäure-Oligomer nach einem der Ansprüche 1 bis 5 und Hilfsmittel.

8. Arzneimittelformulierung nach Anspruch 7, wobei das Arzneimittel in für die orale, rektale, transdermale, dermale, ophtalmologische, nasale, pulmonale oder parenterale Applikation geeigneter Form vorliegt.

9. Arzneimittelformulierung nach Anspruch 7, worin das Arzneimittel in Form von Tabletten, Dragees, Kapseln, Granulat, Trinklösungen, Liposomen, Nanopartikeln, Nanokapseln, Mikrokapseln, Mikrotabletten, Pellets oder Pulvern sowie in Kapseln gefülltem Granulat, in Kapseln gefüllten Mikrotabletten, in Kapseln gefüllten Pellets, in Kapseln gefüllten Nanopartikeln oder in Kapseln gefülltem Pulver vorliegt.

10. Arzneimittelformulierung nach Anspruch 9, worin das Arzneimittel in Form von Nanopartikeln, Mikropellets oder Mikrotabletten vorliegt, die ggf. in Sachets oder Kapseln abgefüllt sein können.

11. Arzneimittelformulierung nach Anspruch 9, worin die festen oralen Dosisformen mit einer magensaftresistenten Beschichtung versehen sind.

12. Arzneimittelformulierung nach einem der Ansprüche 7 bis 11, das eine Menge an Fumarsäure-Oligomer enthält, entsprechend 10 bis 500 mg Fumarsäure.

13. Verwendung nach Anspruch 6 zur Herstellung eines Arzneimittels
(1) zur Therapie einer Autoimmunerkrankung ausgewählt aus der Gruppe bestehend aus der Polyarthritis, Multiplen Sklerose, Graft-versus-Host-Reaktionen, dem juvenilen Diabetes, der Hashimoto-Tyreoiditis, der Grave's disease (Graves Krankheit oder Basedow Krankheit), dem systemischen Lupus erythematodes (SLE), dem Sjögren Syndrom (Sjogren's Syndrome), der perniziösen Anämie und der chronischen aktiven (= lupoiden) Hepatitis,
(2) zur Verwendung in der Transplantationsmedizin,
(3) zur Therapie mitochondrialer Erkrankungen, ausgewählt aus der Gruppe bestehend aus dem Parkinson-Syndrom, der Alzheimer-Krankheit, der Chorea-Huntington-Krankheit, der Retinopathia pigmentosa oder mitochondrialen Enzephalomyopathien, sowie
(4) zur Therapie von NFkappaB vermittelten Erkrankungen, ausgewählt aus der Gruppe, bestehend aus der progressiven systemischen Sklerodermie, der Osteochondritis syphilitica (Wegener's Disease), der Cutis marmorata (Livedo Reticularis), der Behcet-Disease, Panarteritis, Colitis ulcerosa, Vasculitis, der Osteoarthritis, Gicht, Ateriosklerosis, der Reiter's Erkrankung, der bronchozentischen Granulomatose, Encephalitis-Typen, dem Endotoxin-Schock (septisch-toxischer Schock), der Sepsis, der Pneumonie, der Encephalomyelitis, der Anorexia nervosa, der Hepatitis (der akuten Hepatitis, der chronischen Hepatitis, der toxischen Hepatitis, der Alkoholhepatitis, der viralen Hepatitis, der Gelbsucht, der Leberinsuffizienz und der cytomegaloviralen Hepatitis), der Rennert T Lymphomatosis, der mesangialen Nephritis, der Postangioplastie-Restenose, das Reperfusionssyndrom, der cytomegaloviralen Retinopathie, Adenoviralen Erkrankungen wie adenoviralen Erkältungserkrankungen, dem adenoviralen Pharyngoconjunctivalfieber, und der adenoviralen Ophthalmie, AIDS, dem Guillain-Barre-Syndrom, der postherpetischen oder postzoster Neuralgie, der inflammatorischen demyelinisierenden Polyneuropathie, der Mononeuropathia multiplex, der Mukoviszidose, Morbus Bechterew, Barett-Ösophagus, EBV-(Epstein-Barr-Virus)-Infektion, dem kardialen Remodeling, interstitiellen Zystitis, Diabetes mellitus Typ II, der Strahlensensibilisierung maligner Tumore, der Mehrfachresistenz maligner Zellen auf Chemotherapeutika, Granuloma annulare und Krebserkrankungen wie Mamma Karzinom, Kolonkarzinom, Melanom, primäres Leberzellkarzinom, Adenokarzinom, Kaposi Sarkom, Prostatakarzinom, Leukämie wie der akuten myeloischen Leukämie, dem multiplen Myelom (Plasmozytom), Burkitt-Lymphom, und den Castleman-Tumor.

## Claims

1. A carbocyclic oligomer for use as a medicament, wherein the carbocyclic oligomer consists of two units derived from fumaric acid or esters thereof as repeating units, obtainable by polymerisation of the C-C double bonds of the units and according to the following formula (II): 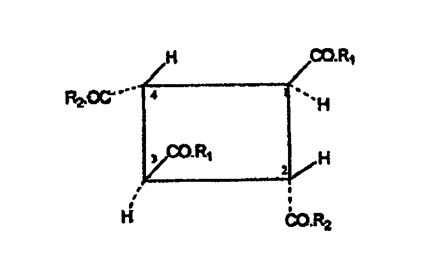 wherein groups R₁ and R₂ are the same or different and are selected from the group consisting of alcohol groups (-OR⁵), and
group R⁵ is selected from hydrogen and C₁₋₂₄ alkyl groups.

2. A carbocyclic oligomer for use as a medicament, wherein the carbocyclic oligomer consists of two units derived from fumaric acid or esters thereof as repeating units, obtainable by polymerisation of the C-C double bonds of the units and according to the following formula (II): 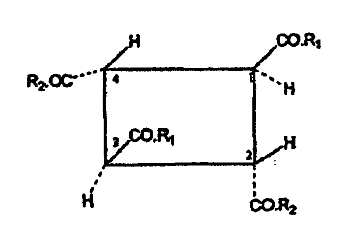 wherein groups R₁ and R₂ are the same or different and are selected from the group consisting of alcohol groups (-OR⁵), and
group R⁵ is selected from the group consisting of methyl, ethyl, n-propyl, isopropyl, n-butyl, sec-butyl, t-butyl, pentyl, cyclopentyl, 2-ethylhexyl, hexyl, cyclohexyl, heptyl, cycloheptyl, octyl, 2-hydroxyethyl, 2- or 3-hydroxypropyl, 2,3-dihydroxypropyl, 2-methoxyethyl, methoxymethyl and 2- or 3-methoxypropyl.

3. The oligomer according to claim 1, wherein R₁ and R₂ each independently represent an alkoxy or hydroxyl group, but both cannot be -OH together.

4. The oligomer according to claim 3, wherein R₁ and R₂ are independently selected from the group consisting of methoxy, ethoxy, propoxy, isopropoxy, n-butoxy, sec-butoxy, tert-butoxy and pentoxy.

5. The oligomer according to claim 3, wherein both R₁ and R₂ are methoxy.

6. A use of a carbocyclic oligomer according to one of the preceding claims for the production of a medicament, wherein the medicament is for treatment of an autoimmune disease, in the transplantation medicine, for treatment of mitochondrial diseases and of NFkappaB mediated diseases.

7. A medicament formulation, containing a fumaric acid oligomer according to one of claims 1 to 5 and an adjuvant.

8. The medicament formulation according to claim 7, wherein the medicament is in a form which is suitable for oral, rectal, transdermal, dermal, ophthalmologic, nasal, pulmonary or parenteral administration.

9. The medicament formulation according to claim 7, wherein the medicament is in the form of tablets, dragées, capsules, granules, drink solutions, liposomes, nanoparticles, nanocapsules, microcapsules, microtablets, pellets or powders as well as granules filled in capsules, microtablets filled in capsules, pellets filled in capsules, nanoparticles filled in capsules or powder filled in capsules.

10. The medicament formulation according to claim 9, wherein the medicament is in the form of nanoparticles, micropellets or microtablets which may optionally be filled in sachets or capsules.

11. The medicament formulation according to claim 9, wherein the oral solid dosage forms are enteric-coated.

12. The medicament formulation according to one of claims 7 to 11 containing an amount of a fumaric acid oligomer which corresponds to 10 to 500 mg of fumaric acid.

13. The use according to claim 6 for the preparation of a medicament
(1) for the therapy of an autoimmune disease selected from the group consisting of polyarthritis, multiple sclerosis, graft-versus-host reactions, juvenile-onset diabetes, Hashimoto's thyroiditis, Grave's disease (Basedow disease), systemic Lupus erythematodes (SLE), Sjogren's syndrome, pernicious anaemia and chronic active (= lupoid) hepatitis;
(2) for use in transplantation medicine;
(3) for the therapy of mitochondrial diseases selected from the group consisting of Parkinson syndrome, Alzheimer's disease, Chorea Huntington disease, retinopathia pigmentosa or forms of mitochondrial encephalomyopathy; as well as
(4) for the therapy ofNF-kappaB mediated diseases selected from the group consisting of progressive systemic sclerodermia, osteochondritis syphilitica (Wegener's disease), cutis marmorata *(livedo reticularis),* Behcet disease, panarteriitis, colitis ulcerosa, vasculitis, osteoarthritis, gout, arteriosclerosis, Reiter's disease, pulmonary granulomatosis, types of encephalitis, endotoxic shock (septic-toxic shock), sepsis, pneumonia, encephalomyelitis, anorexia nervosa, hepatitis (acute hepatitis, chronic hepatitis, toxic hepatitis, alcohol-induced hepatitis, viral hepatitis, jaundice, liver insufficiency and cytomegaloviral hepatitis), Rennert T-lymphomatosis, mesangial nephritis, post-angioplastic restenosis, reperfusion syndrome, cytomegaloviral retinopathy, adenoviral diseases such as adenoviral colds, adenoviral pharyngoconjunctival fever and adenoviral ophthalmia, AIDS, Guillain-Barré syndrome, post-herpetic or post-zoster neuralgia, inflammatory demyelinising polyneuropathy, mononeuropathia multiplex, mucoviscidosis, Bechterew's disease, Barett oesophagus, EBV (Epstein-Barr virus) infection, cardiac remodeling, interstitial cystitis, diabetes mellitus type II, radiosensitisation of malignant tumours, multi-resistance of malignant cells to chemotherapeutic agents (multipharmaceutical preparation resistance in chemotherapy), granuloma annulare and cancers such as mamma carcinoma, colon carcinoma, melanoma, primary liver cell carcinoma, adenocarcinoma, kaposi's sarcoma, prostate carcinoma, leukaemia such as acute myeloid leukaemia, multiple myeloma (plasmocytoma), Burkitt lymphoma and Castleman tumour.

## Revendications

1. Oligomère carbocyclique à utiliser comme agent pharmaceutique, l'oligomère carboxyclique consistant en 2 unités dérivées d'acide fumarique ou de ses esters, en tant qu'unité récurrente, obtenu par polymérisation des doubles liaisons C-C des unités et correspondant à la formule (II) suivante : 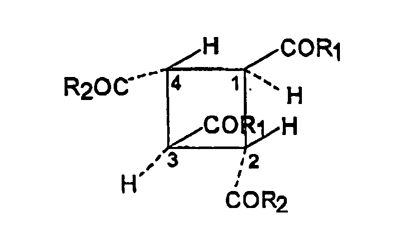
- où les restes R₁ et R₂ sont identiques ou différents et sont choisis parmi le groupe consistant en des restes alcool (-OR⁵) et le reste R⁵ est choisi parmi l'hydrogène et des radicaux alcoyle en C₁₋₂₄.

2. Oligomère carbocyclique à utiliser comme agent pharmaceutique, l'oligomère carboxyclique consistant en 2 unités dérivées d'acide fumarique ou de ses esters, en tant qu'unité récurrente, obtenu par polymérisation des doubles liaisons C-C des unités et correspondant à la formule (II) suivante : 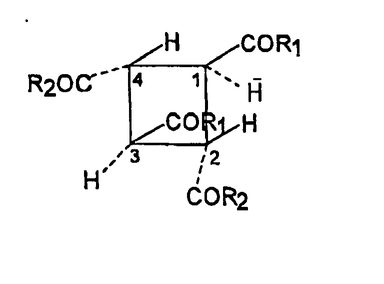 où les restes R₁ et R₂ sont identiques ou différents et sont choisis parmi le groupe consistant en des restes alcool (-OR⁵) et le reste R⁵ est choisi parmi le groupe consistant en méthyle, éthyle, n-propyle, isopropyle, n-butyle, s-butyle, t-butyle, pentyle, cyclopentyle, 2-éthylhexyle, hexyle, cyclohexyle, heptyle, cycloheptyle, octyle, 2-hydroxyéthyle, 2- ou 3-hydroxypropyle, 2,3-dihydroxypropyle, 2-méthoxyéthyle, méthoxyméthyle et 2- ou 3-méthoxypropyle.

3. Oligomère selon la revendication 1, où R₁ et R₂ représentent indépendamment, un reste alcoxy ou hydroxy, mais les deux ne peuvent pas être -OH.

4. Oligomère selon la revendication 3, où R₁ et R₂ sont choisis indépendamment l'un de l'autre, parmi le groupe consistant en méthoxy, éthoxy, propoxy, isopropoxy, n-butoxy, s-butoxy, t-butoxy et pentoxy.

5. Oligomère selon la revendication 3, où R₁ et R₂ représentent tous deux méthoxy.

6. Utilisation d'un oligomère carbocyclique selon l'une des revendications précédentes, pour la préparation d'un agent pharmaceutique, où l'agent pharmaceutique est destiné au traitement d'une maladie auto-immune, dans la médecine des transplantations, pour le traitement de maladies mitochondriales et pour les maladies à médiation par le NFkappaB.

7. Formulation d'agent pharmaceutique contenant un oligomère d'acide fumarique selon l'une des revendications 1 à 5, et un auxiliaire.

8. Formulation d'agent pharmaceutique selon la revendication 7, où l'agent pharmaceutique est présent sous une forme appropriée pour une administration orale, rectale, transdermique, dermique, ophtalmologique, nasale, pulmonaire ou parentérale.

9. Formulation d'agent pharmaceutique selon la revendication 7, où l'agent pharmaceutique est présent sous forme de comprimés, dragées, capsules, granulé, solutions buvables, liposomes, nanoparticules, nanocapsules, microcapsules, microcomprimés, pellets ou poudres, ainsi que de granulé dans des capsules, microcomprimés dans des capsules, pellets dans des capsules, nanoparticules dans des capsules ou poudre dans des capsules.

10. Formulation d'agent pharmaceutique selon la revendication 9, où l'agent pharmaceutique est présent sous forme de nanoparticules, micropellets ou microcomprimés, qui peuvent être disposés le cas échéant, dans des sachets ou des capsules.

11. Formulation d'agent pharmaceutique selon la revendication 9, où les formes de dosage oral solides sont munies d'un enrobage gastro-insoluble.

12. Formulation d'agent pharmaceutique selon l'une des revendications 7 à 11, qui contient une quantité d'oligomère d'acide fumarique, correspondant à 10 à 500 mg d'acide fumarique.

13. Utilisation selon la revendication 6, pour la préparation d'un agent pharmaceutique
(1) pour la thérapie de maladies auto-immunes sélectionnées parmi le groupe consistant en la polyarthrite, la sclérose en plaques, les réactions du greffon contre l'hôte, le diabète juvénile, la thyroïdite de Hashimoto, la maladie de Grave (ou maladie de Basedow), le lupus érythémateux disséminé (SLE), le syndrome de Sjögren (Sjogren's Syndrome), l'anémie pernicieuse et l'hépatite chronique active (= lupoïde),
(2) pour une utilisation en médecine des transplantations,
(3) pour la thérapie des maladies mitochondriales, sélectionnées parmi le groupe consistant en le syndrome de Parkinson, la maladie d'Alzheimer, la chorée de Huntington, la rétinopathie pigmentaire ou les encéphalomyopathies mitochondriales, ainsi que
(4) pour la thérapie de maladies à médiation par le NFkappaB, sélectionnées parmi le groupe consistant en la sclérodermie systémique progressive, l'ostéochondrite syphilitique (maladie de Wegener), la cutis marmorata (livedo reticularis), la maladie de Behçet, la panartérite, la colite ulcérosante, la vasculite, l'ostéoarthrite, la goutte, l'artériosclérose, la maladie de Reiter, la granulomatose bronchocentrique, des types d'encéphalite, le choc endotoxique (choc septique toxique), la septicémie, la pneumonie, l'encéphalomyélite, l'anorexie, l'hépatite (hépatite sévère, hépatite chronique, hépatite toxique, hépatique alcoolique, hépatite virale, jaunisse, insuffisance hépatique et hépatite cytornégalovirale), la lymphomatose T de Rennert, la glomérulonéphrite chronique, la resténose post-angioplastie, le syndrome de reperfusion, la rétinopathie cytomégalovirale, les maladies adénovirales, comme les maladies adénovirales de refroidissement, la fièvre adénovirale de la pharyngoconjonctive, et l'ophtalmie adénovirale, le SIDA, le syndrome de Guillain-Barré, les névralgies post-herpétiques ou post-zona, la polyneuropathie démyélinisante inflammatoire, la mononeuropathie multiple, la mucoviscidose, la maladie de Bechterew, l'oesophage de Barrett, une infection par le virus d'Epstein-Barr, la remodélisation cardiaque, la cystite interstitielle, le diabète de type II, la sensibilisation au rayonnement des tumeurs malignes, la résistance multiple des cellules malignes aux composés chimiothérapeutiques, le granulome annulaire et des maladies cancéreuses comme le carcinome mammaire, le carcinome du colon, le mélanome, le carcinome primaire des cellules hépatiques, l'adénocarcinome, le sarcome de Kaposi, le carcinome de la prostate, les leucémies comme la leucémie myéloïde sévère, le myélome multiple (plasmocytome), le lymphome de Burkitt et la tumeur de Castelman.
